# EUROPEAN PATENT APPLICATION

(11) **EP 2 060 288 A1**
(43) Date of publication of application: **20.05.2009**
(21) Application number: 07021985.2
(22) Date of filing: 13.11.2007
(51) Int. Cl.: A61M 5/315, H01H 15/00, H01H 19/20, A61M 5/31

(54) **Administration device having input unit**

(71) Applicant: F.HOFFMANN-LA ROCHE AG, 4070 Basel (CH); Roche Diagnostics GmbH, 68305 Mannheim (DE)
(72) Inventor: Blasise, Mouline, 30700 La Capelle-Masmolene (FR); Remde, Axel, 3432 Lützelflüh-Goldbach (CH); Heiniger, Hanspeter, 4932 Lotzwil (CH); Blasberg, Peter, 69469 Weinheim (DE); Koehler, Matthias, 69514 Laudenbache (DE); Rasch-Menges, Juergen, 69514 Schwetzingen (DE)
(74) Representative: Poredda, Andreas

(57) **Abstract**

Administration device comprising a device housing (10) and an input unit, the input unit comprising a control element (50), the control element (50) being arranged, at least in part, outside housing (10) and being movable by a first motion (A) with respect to the housing (10) into either of at least two selection positions, the control element (50) being further movable by a second motion (B) with respect to the housing (10) into either of at least two elongation positions, wherein the motion direction of the first motion (A) is different from the motion direction of the second motion (B).

## Description

The following invention is related to an administration device for the self-administration of liquid drugs in adjustable doses. Administration devices according to the present invention may especially be used for the self-administration of insulin in the framework of diabetes therapies, but may also be used for the administration of other liquid drugs such as pain relievers or growth hormones.
In the therapy of insulin-dependent diabetes mellitus, pen-shaped injection devices for the self-administration of insulin in adjustable doses are widely used and well known in the art, such as the device disclosed in WO 93/16740.
In many cases, a state-of-the-art diabetes therapy also requires frequent self measurements of blood glucose and careful therapy recording, the therapy records including both glucose measurements and insulin administrations as well as additional therapy related data concerning intake of food, namely carbohydrate amounts and potentially sportive activity, illnesses, intake of additional drugs and so on. Appropriate insulin dosing is potentially dependent on all of these factors. It is a well recognized problem that therapy recording is cumbersome and therapy records are incomplete, erroneous or completely missing in many cases.
In US 6585698 a pen-type injector is disclosed, the injector having a control element is in a first state engage the manually operated drive mechanism for setting and administering insulin doses. In a second state, the control element is disengaged from the drive and may be used for entering parameters or additional data. The selective engagable control element however, results in a complex and costly design.
In US 5536249 a pen-type injector is disclosed, the injector being capable of storing the amount of administered drug doses along with additional information, especially time and date of administration, in an internal memory and download the data stored in the external memory to an external device. However, no input unit for comfortable entry of further therapy-related data is provided.
It is the objective of the present invention to provide administration devices for the self-administration of liquid drugs in adjustable doses which comprise an input unit and are both simple and intuitive in use while cost efficient in manufacture.
An administration device according to the present invention comprises a manually driven dosing mechanism, a drug reservoir and a dose setting means, the dose setting means being coupled to the dosing mechanism. The administration device further comprises an electronics module and a housing, the housing comprising the dosing mechanism, the electronics module and the drug reservoir. The housing further defines a device axis and is adapted to be held in a hand for drug administration. The administration device may especially be pen-shaped.
An administration device according to the present invention further comprises an input unit, the input unit being coupled to the electronics module. The input unit comprises a control element, the control element being different from the dose setting means, the control element being arranged, at least in part, outside the housing. The control element is movable by a first motion with respect to the housing into either of at least two selection positions, and the control element is further movable by a second motion with respect to housing into either of at least two elongation positions, wherein the motion direction of the first motion is different from the motion direction of the second motion. The control element is adapted to be manually operated by the user.
In preferred embodiments, the first motion is a linear motion, the linear motion being substantially parallel with the device axis. In alternative embodiments, the first motion is a different type of motion, such as linear motion in a direction which is not substantially parallel with the device axis or a non-linear translation motion or a rotational motion or a combination of such. In especially preferred embodiments, the second motion (B) is a rotation, the axis of rotation of the second motion is defined by the device axis.
For his kind of embodiment, the housing is preferably of substantially cylindrical shape or consists of multiple substantially cylindrical and coaxial segments.In especially preferred embodiments, the control element substantially has the form of a preferably curled cylindrical ring, the ring surrounding a portion of the housing, wherein the housing outer shell defines a sliding surface for control element, such that the control element may slide on the housing outer shell along the device axis into either of the alt least two selection positions and may rotate about the device axis into either of the at least two elongation positions. The dimension of the control element along the device axis is preferably considerably smaller than the dimension of the administration device along the device axis.
In alternatively preferred embodiments, the first motion is a first linear motion, the first linear motion being substantially parallel with the device axis, while the second motion is a second linear motion with the second linear motion being substantially transversal to the first linear sliding motion.
In preferred embodiments, at least one of the selection positions is a catching position. In especially preferred embodiments, more than one selection position is a catching position. In some embodiments, all selection positions are catching positions. The catching property may be realized by spring elements, such as springy hooks provided at either of the control element or the housing interacting with corresponding cutouts and/or stoppers at the other of the control element or the housing. The at least one spring element results in a catching force being exerted onto a catching force transmitter, the catching force transmitter being comprised by or being coupled to the control element, if the control element is in a position different from a catching position. The catching force drives the control element into a neighboring catching position.
In preferred embodiments, the first motion of the control element is enabled only if the control element is in a defined neutral elongation position. In preferred embodiments, the second motion of the control element is enabled only if the control element is in either of the at least two selection positions. In especially preferred embodiments, the input unit comprises a guide, the guide restricting the motion of the control element. In especially preferred embodiments, the guide is defined by an aperture in the housing, such that the motion of the control element is restricted by the interaction of a motion restrictor with the aperture, the motion restrictor being comprised by or being coupled to the control element, the motion restrictor projecting through the aperture. The motion restrictor may, e.g., have the form of a pin, the pin projecting through the aperture into the housing.
In preferred embodiments, moving the control element into either of at least two elongated operation positions, the elongated operation positions being different from a neutral elongation position, results in a first restoring force being exerted onto the control element. In especially preferred embodiments, the second motion is a rotation and exerting the first restoring force onto the control element results in a first restoring moment being exerted onto the control element.
In especially preferred embodiments comprising the exertion of a first restoring force onto the control element, the input unit comprises an spring element, the spring element being arranged inside the housing and in symmetric alignment with the device axis. The spring element is preferably supported by supporting elements, resulting in the spring element being substantially fixed in the housing. The supporting elements are preferably realized as pockets, the pockets being integral with and inside the housing. In this kind of embodiment, moving the control from the neutral elongation position into an elongated operation position results in the spring element exerting the first restoring force onto the control element. The spring element may, e.g., be substantially U-shaped, such that the elongated legs of the spring elements exert the first restoring force onto a restoring force transmitter, the first restoring force transmitter being comprised by or coupled to the control element, the first restoring force transmitter being, at least in part, located between the elongated legs of the spring element. The first restoring force transmitter may, e.g., have the form of a pin, the pin projecting into the housing.
In especially preferred embodiments comprising an elongated, e.g., U-shaped, spring element and at least one catching position, the spring element is adapted for exerting both a catching force and a first restoring force onto the control element, the spring element additionally defining the at least one catching position. For this purpose, the spring element may comprises at least one salient along its length with the first restoring force transmitter being, at least in part, located between the elongated legs of the spring element. This arrangement results in substantially no axial force being exerted by the spring element onto the restoring force force transmitter if the operation element is aligned with and projects through either of the at least one salient, resulting in a stable position of the control element. If the operation element is not aligned with and does not project through either of the at least one salient, a significant catching force is exerted by the spring element onto the restoring force transmitter. In this kind of embodiment, the first restoring force transmitter additionally serves as catching force transmitter.
In alternative embodiments, more than one spring element may be used. In further alternative embodiments, other types of spring elements, such as one or multiple compression springs and/or extending springs and/or bending springs are provided the at least one spring interacting with the control element and the housing. The spring element may also be made from plastics and may especially be integral with the housing and/or the control element.
In preferred embodiments, the control element comprises an operation element, the operation element projecting into the housing. The operation element is adapted to transfer at least either of the first motion and the second motion of the control element to receiving elements, wherein the receiving elements may comprise electrical contact elements, switches, potentiometers, or the like. In especially preferred embodiments, the control element substantially has the form of a cylindrical ring or ring section and the operation element is integral with the ring, the operation element projecting in radial direction from the inner shell of the ring into the housing.
In especially preferred embodiments, the preferably present operation element comprises a base member and at least one extension member, with the base member projecting into the housing and the at least one extension member projecting from the base member, the at least one extension member being adapted to operate the receiving elements. In some especially preferred embodiments, the preferably present operation element comprises two extension members, the two extension members projecting substantially transversally from the preferably present base member into opposite directions.

In some preferred embodiments, the preferably present base member of a preferably present operation element serves as at least either of a first restoring force transmitter as motion restrictor, or catching force transmitter. In especially preferred embodiments, the base member serves as first restoring force transmitter, as catching force transmitter and as motion restrictor.
In preferred embodiments, the input unit comprises contact elements, wherein each contact element can be selected by moving the control element into the selection position corresponding to the contact element, and wherein a selected contact element can be operated by moving the control element from a neutral elongation position into an elongated operation position while keeping the selection position.
In especially preferred embodiments, the contact elements, are, at least in part, realized by monostable micro pushbuttons, the monostable micro pushbottons being arranged inside the housing and being operated via the operation element. Besides monostable micro pushbuttons, further types of contact elements, such as bistable pushbuttons an/or switches may be provided. In further embodiments, the contact elements are, at least in part, realized by metallic leads and at least one connection element, the at least one connection element selectively connecting selected leads, depending on the position of the control element. The metallic leads may be provided, e.g., on a Printed Circuit Board (PCB) or by selective metallization of plastic components such as the housing inner shell or the housing outer shell. The connection elements may be realized by at least one metallic member, the metallic member being comprised to or attached by the control element and/or metallic leads, the metallic leads being comprised by the control element.
In preferred embodiments, at least one of the selection positions selects a pair of contact elements, such that a first contact element of a selected pair of contact elements can be operated by moving the control element into a first elongated operation position and in that a second contact element of a selected pair of contact elements can be operated by moving the control element into a second elongated operation position. In especially preferred embodiments, the first elongated operation position and the second elongated operation position are on opposite sides of a neutral elongation position, such that the first contact element and the second contact element of a selected pair of contact elements may be operated by moving the control element from the neutral elongation position into opposite directions. In especially preferred embodiments, more than one of the selection position each selects a pair of contact elements. More particularly, each of the selection positions selects a pair of contact elements in especially preferred embodiments. For this kind of embodiment, the number of selection positions is half the number of contact elements.
In especially preferred embodiments, a first contact element axis and a second contact element axis are provided, the first contact element axis and the second contact elements axis being parallel and equidistant to the device axis. The first contact elements of the pairs of contact elements are arranged in a first line, the first line defining the first contact element axis, while the second contact elements of the pairs of contact elements are arranged in a second line, the second line defining the second contact element axis.
In preferred embodiments, operating a contact element results in a second restoring force being exerted onto the control element. The second restoring force may be exerted by spring elements such as at least one compression spring or leaf spring. The second restoring force is transmitted to the control element via a second restoring force transmitter. In especially preferred embodiments, the contact elements are, at least in part, monostable micro pushbuttons, the monostable micro pushbuttons generating a contact element restoring force when being operated, wherein the second restoring force is the contact element restoring force. In especially preferred embodiments having an operation element, the operation element comprising a base member and at least one extension member, the second restoring force is exerted onto the at least one extension member, the at least one extension member serving as second restoring force transmitter. In especially preferred embodiments, the second motion is a rotation and exerting the first restoring force onto the control element results in a first restoring moment being exerted onto the control element. In especially preferred embodiments, both a first restoring force and a second restoring force are exerted onto the control element. In alternative embodiments, only a first restoring force or only a second restoring force is exerted onto the control element. In further embodiments, no restoring momentum is exerted onto the control element.
In preferred embodiments, the housing comprises a first housing component and a second housing component. The first housing component and the second housing component are substantially non-overlapping along the device axis for especially preferred embodiments. In especially preferred embodiments, the housing further comprises of at least two substantially cylindrical segments, the cylindrical segments being coaxial and having substantially equal diameters, such that the housing is, at least in part, substantially cylindrical in the assembled state.
In especially preferred embodiments, the housing further comprises a first intermediate housing component and a second intermediate housing component. The first intermediate housing component and the second intermediate housing component are arranged between the first housing component and the second housing component along the device axis and may show an overlap with first the housing component and/or the second housing component. In combination, the first intermediate housing component outer shell and the second intermediate housing component outer shell define a sliding surface for the control element. The first intermediate housing component outer shell and the second housing component outer shell may especially be half cylindrical, and be combined to form a substantially cylindrical element.
In especially preferred embodiments comprising a substantially cylindrical housing, the first housing component is coupled to the first intermediate housing component and to the second intermediate housing component via snap-on connections. In the same way, the second housing component is coupled to first intermediate housing component and to the second intermediate housing component via snap-on connections. The control element surrounds at least a portion of the first intermediate housing component outer shell and the second intermediate housing component outer shell, such that the control element exerts a radial force onto the first intermediate housing component and onto the second intermediate housing component, resulting in the first intermediate housing component and the second intermediate housing component being fixed with respect to each other. In alternative embodiments, the first intermediate housing component and the second intermediate housing component are fixed with respect to each other by specialized fixing means, such as additional snap-on-connections.
In further alternative embodiments, only one intermediate housing component may be provided. Such intermediate housing components may especially have an cylindrical outer shell, defining a sliding surface for the control element or more than two intermediate housing components may be provided.
In preferred embodiments, the drug reservoir is a cylindrical cartridge, the cartridge axis being substantially parallel with the device axis, the cartridge comprising a plunger, the plunger being displacable in a direction defined by the cartridge axis, and the dosing mechanism comprises a piston rod, the piston rod being coaxially aligned with the cartridge axis and being in contact with the plunger, such that the plunger is displaced by a displacement distance Δ in a direction defined by the cylinder axis if the piston rod is displaced by a displacement distance Δ, wherein the displacement distance Δ is controlled by a dose setting means (25). The dosing mechanism may be design, e.g. according to the disclosure in WO 93/16740 or other suited design known in the art.
In preferred embodiments, at least one of the selection positions is a numeric value entry position, such that moving the control element from a neutral elongation position into a first elongated operation position increases a numeric value to be entered by at least one increment I and that moving the control element from a neutral elongation position into a second elongated operation position decreases a numeric value to be entered by at least one increment I, wherein the control element is in the at least one numeric value entry position. In the framework of diabetes, therapy, the numeric values which may be entered via the input unit preferably comprise therapy related data such as amounts and/or blood glucose values.
In especially preferred embodiments, at least two of the selection positions are numeric value entry position, such that the increment I equals a first increment 11 if the control element is in a first numeric value entry position and that the increment I equals a second increment 12 if the control element 50 is in a second numeric value entry position, wherein the first increment I1 is different from the second increment I2.
In preferred embodiments, the selection positions comprise an entry completing position such that any input made via the control element with the control element being in an selection position different form the entry completing position may be accepted by moving the control element from a neutral elongation position into a first elongated operation position with the control element being in the entry completing position and any input made via the control element with the control element being in an selection position different form the entry completing position may be canceled by moving the control element from its neutral elongation position into a second elongated operation position with the control element being in the entry completing position. In alternative embodiments, no dedicated entry completing position is provided but the entry is assumed to be complete if the data to be entered are not altered for a given time of e.g., 5 seconds.
In preferred embodiments, the electronics module comprises memory, the memory being adapted to store data entered via the input unit. The memory may be of several kinds known in the art, such as an Electrically Erasable Read Only Memory (EEPROM) or a Dynamic Random Access Memory (DRAM) but is preferably a Static Random Access Memory (SRAM) or Flash Random Access Memory (FRAM).
In especially preferred embodiments, the electronics module further comprises a data interface, the data interface being adapted to download data stored in the memory to an external device. In some preferred embodiments, the data interface comprises wireless interface means such as a BLUETOOTH Radio Frequency (RF) interface and/or a serial infrared interface, for example according to the IRDA standard. In further preferred embodiments, the data interface comprises a wired interface such as a Universal Serial Bus USB interface and/or a standard serial interface according RS232. A wired interface providing a power supply, such as USB, may additionally be used for recharging a preferably present power supply. In especially preferred embodiments, the data interface is bidirectional, but it may also be unidirectional and be adapted only for transmitting data in alternative embodiments.
Preferably the electronics module has a unique electronics module identification code such as a serial number stored therein and the electronics module identification code is downloaded along with the data stored in the memory. Transmitting an unique identification code allows an external device to identify the administration device and allows to download data from multiple administration devices without the data being mismatched. Accordingly the same patient may use multiple devices in parallel, e.g. devices for different drugs and/or he may have different administration devices for one drug, e.g. one at home and another one at his working place. For example a state-of-the-art-therapy of diabetes typically requires the administration of at least two types of insulin, a fast acting bolus insulin for covering carbohydrate intake and for correcting undesirably raised blood glucose values, and a long acting insulin covering the patient's basal insulin demand. Here it is imprtant not to mismatch the administered doses of the different types of insulin. When data download is being performed by a healthcare professional, the identification code may be used for patient identification purposes.
In preferred embodiments, the electronics module comprises a dose calculator, the dose calculator being adapted to calculate a dose amount of the liquid drug based, at least in part, on data entered via the input unit. In the framework of diabetes therapy, the dose calculator may calculate insulin dose amounts based on a first set of patient-specific and/or time-of-day dependent factors, the first set of factors specifying the dose to cover a given carbohydrate amount. In the framework of diabetes therapy, a second set of patient-dependent and/or time-of-day dependent factors, the second set of factors specifying the dose amount required to correct a given raise in blood glucose, may be used by the dose calculator in order to calculate an appropriate correction dose amount of insulin. In some embodiments, the administration device comprises determining means such as a blood glucose meter, the determining means being part of the electronics module or coupled to the electronics module.
In the framework of diabetes therapy, the electronics module comprises a carbohydrate amount database in especially preferred embodiments. The carbohydrate amount database is preferably coupled to the preferably present dose calculator, the carbohydrate amount database storing the relative and/or absolute carbohydrate amounts of a number of meals, such that meals may be selected from the carbohydrate amount database via the control element. Such a database allows for a simplified entry of typical and/or frequently taken meals of defined composition.
In preferred embodiments, the electronics module comprises a display, the display being adapted to show data entered via the input unit. The display may especially be a numeric or alphanumeric display, such as a Liquid Crystal Display (LCD). Besides data entered via the control element, the display may be adapted to display other information such as a date or time-of-day information generated by a preferably present clock circuit. In especially preferred embodiments, the display is further capable of displaying several selectable menu items and/or functions which may be selected via the data entry means. In especially preferred embodiments, the display is further adapted to show dose amounts, such as dose amounts which are set for administration via the dose setting means or dose amounts which are calculated by the preferably present dose calculator. In especially preferred embodiments, the electronics module further comprises additional indication and/or data out put means such as a buzzer and/or a pager vibrator.
In preferred embodiments, the electronics module comprises a power supply which may comprise a single use battery, a rechargeable battery, a capacitor, a solar cell, or the like, or any combination of those. Depending on factors such as the power consumption, the power supply capacity and the power supply lifetime in relation to the electronics module lifetime, the power supply may or may not be replacable.
In preferred embodiments, the electronics module comprises a controller, the controller being preferably realized as Application Specific Integrated Circuit (ASIC) but may also have the form of a standard micro controller, discrete electronics, or the like. Further components of the electronics module may be integral with the controller.

In preferred embodiments, the electronics module comprises a clock circuit and the memory stores a time stamp together with administered doses and with therapy related data entered via the data entry means. The time stamps generated by the clock circuit preferably comprises a date portion and a time-of-day portion, with the resolution being stored with a resolution of one minute or one second.
The administration of insulin in the framework of diabetes therapy is dependent on therapy related data, especially carbohydrate amounts and/or blood glucose values. However an administration device according to the present invention allows for dose setting and administration without prior entry of corresponding therapy related data. In preferred embodiments, a warning is therefore given to the user via the preferably present display and/or additional indication means if no corresponding therapy related data are being entered or have been entered within a given timeframe ol" e.g., 2 minutes before or after drug administration.
Therapy related data may be entered via the input unit without corresponding drug administration. While this may happen on intention in some cases it is also known to happen mistakenly. Accordingly, a warning is preferably given to the user via the preferably display and/or additional indication means if a drug dose is not being administered or has been administered within a given timeframe, of, e.g., 2 minutes before or after entering data normally accompanied by drug administration.
Besides downloading data stored in the preferably present memory, the preferably present data interface may be used to upload data from an external device, such as a PC or a Personal Digital Assistant (PDA), to the electronics module in especially preferred embodiments. Those data may, for example, contain setup data such as required for time and date setting of the preferably present clock circuit. In the framework of diabetes therapy, those data may further contain a preferably present carbohydrate amount database and/or a first set of patient-specific and/or time-of-day dependent factors specifying the dose to cover a given carbohydrate amount and/or a second set of patient-dependent and/or time-of-day dependent factors specifying the dose required to correct a given raise in blood glucose or the like.

In the following, an exemplary embodiment for an administration device for liquid drugs in adjustable doses is described in greater detail and with reference to the figures. The exemplary device may especially be an administration device for the self administration of insulin.
Figure 1 shows an administration device according to a preferred exemplary embodiment of the present invention.
Figure 2a to 2c show a more detailed view of the control element of the administration device according to Figure 1.
Figure 3 shows an exploded view of the input unit along with additional related components of an administration device according to Figure 1.
Figure 4 shows a schematic detailed view of a section of a printed circuit board ,the printed circuit board being part of an administration device according to Figure 1.
Figure 5 shows a first sectional view of the input unit of an administration device according to Figure 1.
Figure 6 shows a cut-away top view of a section of an administration device according to Figure 1.
Figure 7 shows a second sectional view of the input unit of an administration device according to Figure 1.
Figure 8 shows a detailed view of a control element of an administration device according to Figure 1.
Figure 9 shows a block diagram of the electronics module of the exemplary administration device.

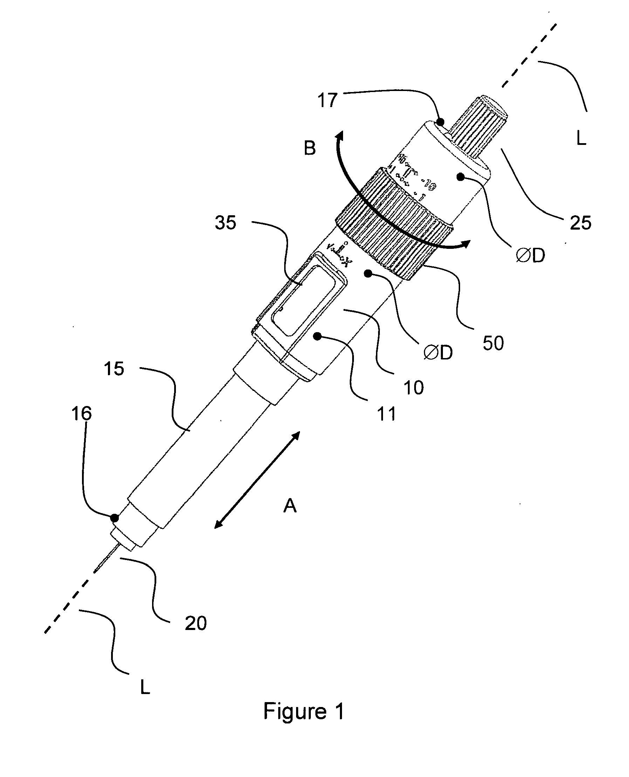

Figure 1 shows an administration device for the self administration of insulin according to a preferred exemplary embodiment of the present invention.
Most components of the administration device are enclosed by a pen-shaped housing 10, the housing 10 enclosing a manually operated dosing mechanism as well as an electronics module (not visible in Figure 1), and a detachable cartridge cover 15, the cartridge cover enclosing a replaceable insulin cartridge. The housing 10 and the cartridge cover 15 are of substantially cylindrical shape and are arranged coaxially, defining a device axis L. A first device front face 16 of the cartridge cover 15 comprises coupling means (not shown) for removably coupling with an injection cannula 20, wherein the injection cannula 20 projects from the cartridge cover 15.
The cannula 20 is typically disposable and should be used for a single injection only for sterility reasons. In alternative embodiments however, the cannula 20 may be used for several injections. A cover (not shown in Fig. 1) is provided to cover and protect the cartridge cover 15, the cannula coupling means and the cannula 20 when the administration device is not in use.
Dose setting knob 25 serves as dose setting means and may project from a second device front face 17, with the second device front face 17 being opposite to the first device front face 16. The dose setting knob 25 is normally in a retracted position inside the housing 10 and may be extended by pressing a release key (on the backside of the housing 10, not visible). In its extended position, the dose setting knob 25 may be rotated in a first direction for increasing the dose amount of a dose to be administered or may be rotated in a second direction for decreasing the dose amount of a dose to be administered, the second direction being opposite to the first direction. The minimum dose amount increment is defined by rotational catching positions of the dose setting knob 25 corresponding to, e.g., 0.5 IU (International Units) while a full rotation of the dose setting knob 25 corresponds to, e.g. 20 IU. After setting the dose amount of a dose to be administered, the patient may grip the administration device with one hand and perform the injection by (i) piercing the skin of, e.g., an upper arm or thigh by moving the administration device into a direction defined by the axis L with the tip of the cannula 20 pointing towards the skin, and (ii) driving the dosing mechanism to administer the insulin dose by slowly pressing the dosing knob 25 down into its retracted position. The manually driven dosing mechanism may be designed according to the disclosure of WO 93/16740 or another suited design known in the art. For clarity reasons, the driving mechanism is generally not shown in the figures.

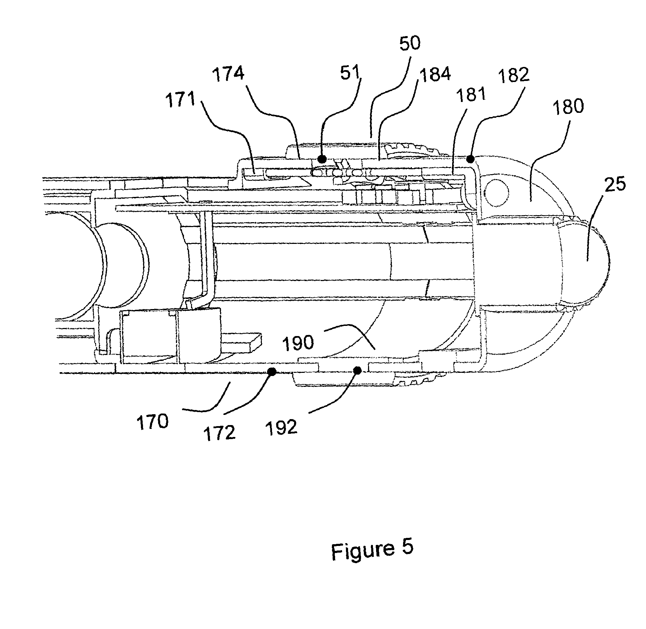

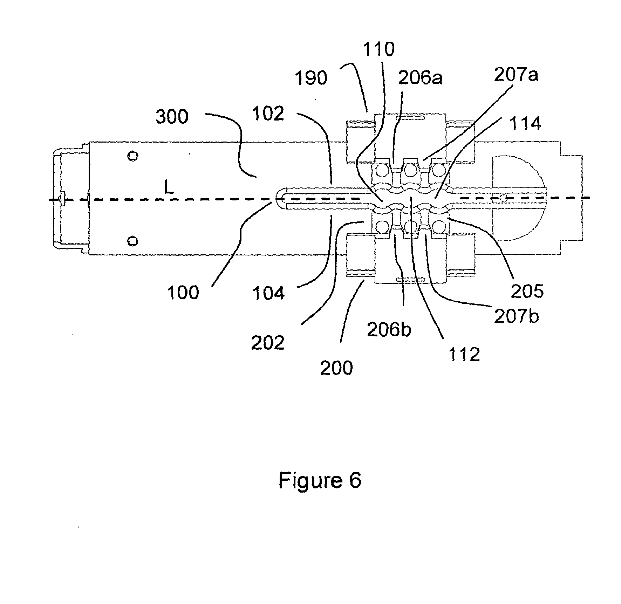

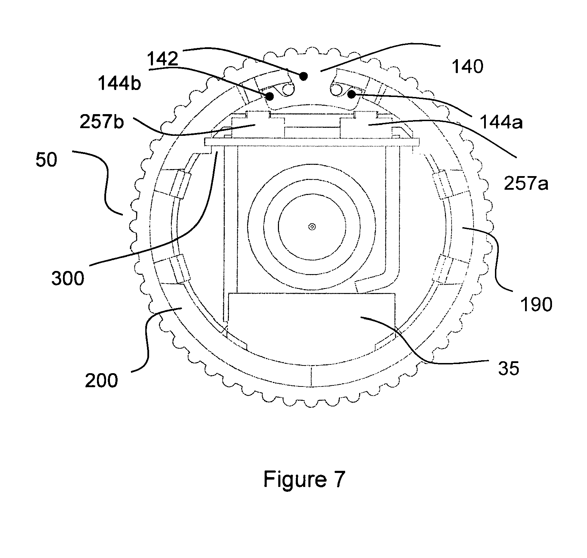

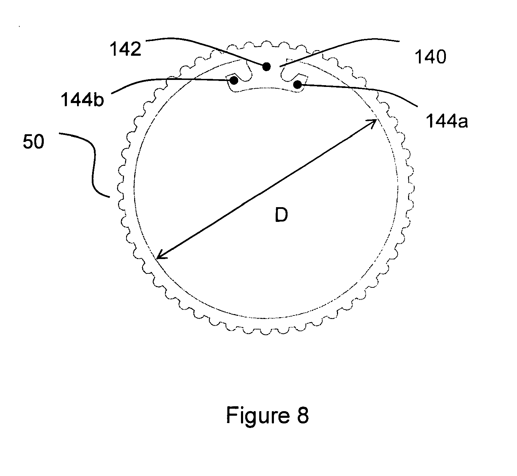

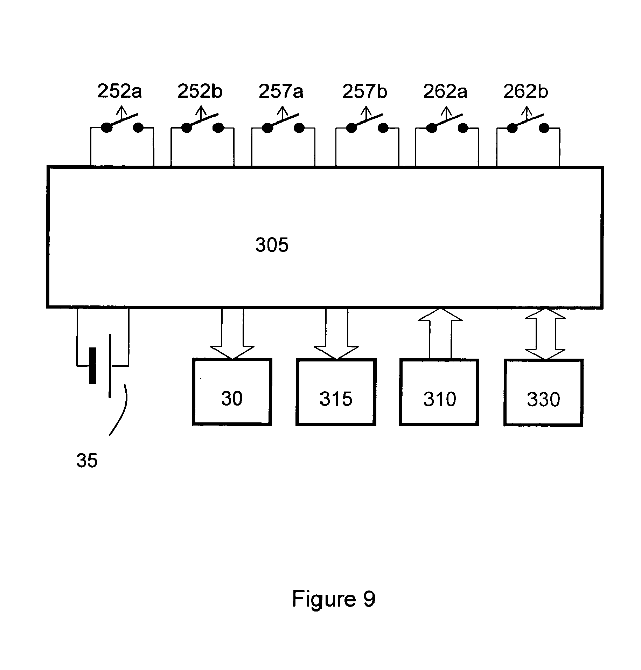

The administration device further comprises a control element 50, the control element 50 being substantially ring-shaped and in coaxial alignment with the housing 10 and surrounding the housing outer shell on the length of the control element 50, the length of the control element 50 being measured along the device axis L. The outer surface of the control element 50 is curled to allow secure gripping.

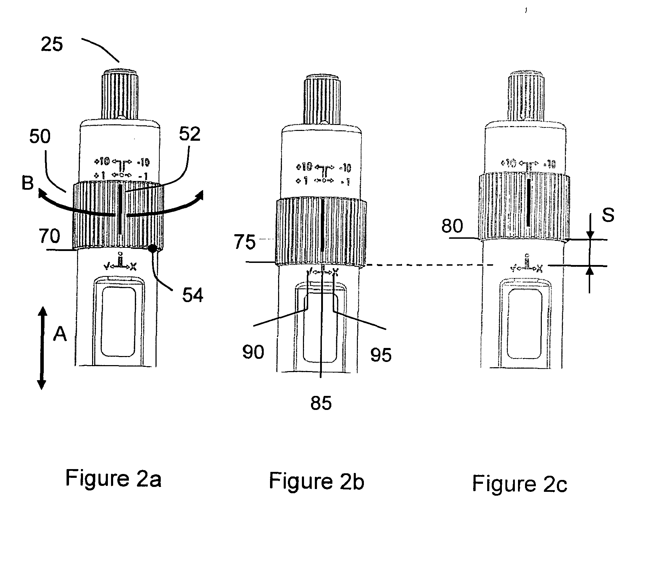

Figure 2a, Figure 2b, and Figure 2c show a more detailed view of the control element 50. The control element 50 may slide on the housing outer shell 11 with a first motion A into either of a first selection position 70, a second selection position 75 and a third selection position 80. The first motion A is a linear motion with the motion direction being substantially parallel to the device axis L. The selection positions 70, 75, 80 are defined by the position of a reference edge 54 of the control element 50 along the device axis L. The control element 50 may further be rotated on the housing outer shell I I from a neutral elongation position 80 into a first elongated operation position 90 or a second elongated operation position, 95, respectively.
Figure 2a shows the control element 50 in the second selection position 75, Figure 2b shows the control element 50 in the first selection position 75 and Figure 2c shows the control element 50 in the third selection position 80, the three selection positions being catching positions. In combination, the first selection position 70 and the third selection position 80 define the travel distance S of the control element 50, the travel distance S being preferably in the range of some millimeters. Figure 2a to 2c show the control element (50) in its neutral elongation position 85. From this neutral elongation position 85, the control element 50 may be rotated by the second motion B either into the first elongated operation position 90 or into the second elongated operation position 95. The corresponding rotational angles are preferably in the range of some degrees. The rotational position of the control element 50 is indicated by a reference mark 52 on the control element 50.

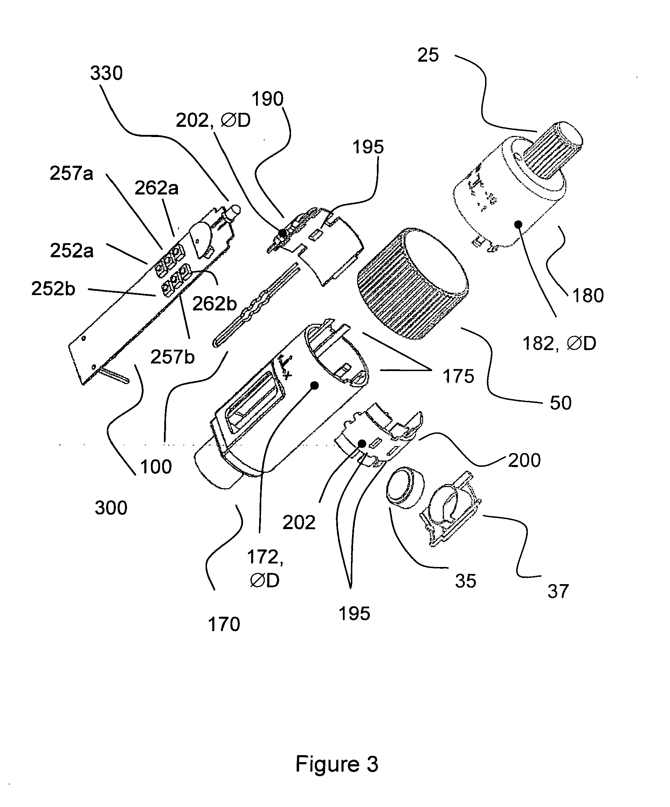

Figure 3 shows an exploded view of the input unit along with additional related components. The housing 10 comprises a first housing component 170 and a second housing component 180. Both the first housing component outer shell 172 and the second housing component outer shell 182 are substantially cylindrical with equal outer diameters D and in coaxial alignment with the device axis L.
The housing 10 further comprises a first intermediate housing component 190 and a second intermediate housing component 200. The intermediate housing components 190, 200 have half cylindrical outer shells 192, 202 and are arranged between the first housing component 170 and the second housing component 180. In combination, the first intermediate housing component 190 and the second intermediate housing component 200 form a substantially cylindrical segment with the outer diameters D in coaxial alignment with the device axis L. The first housing component 170 and the second housing component 190 are coupled to the first intermediate housing component 190 and to the second intermediate housing component 200 via snap-on connections (175, 195). The snap-on connections (175, 195) are realized by snap-fits 175, the snap-fits 175 projecting from the first housing component 170 and the second housing component 180 and by corresponding cutouts 195 in the first intermediate housing component 190 and the second intermediate housing component, 200, respectively.
Figure 3 further shows six contact elements 252a, 252b, 257a, 257b, 262a, 262b, the contact elements 252a, 252b, 257a, 257b, 262a, 262b serving as receiving elements and being realized as monostable micro pushbuttons.
The contact elements 252a, 252b, 257a, 257b, 262a, 262b as well as the display 35 (see Figure 1, not shown in Figure 3) are part of the electronics module (see Figure 9, not shown in Figure 3) and are arranged along with other electronics components on a Printed Circuit Board (PCB) 300 inside the housing 10 with PCB 300, the PCB forming a plane in parallel alignment with the device axis L. The electronics module is described below in greater detail.

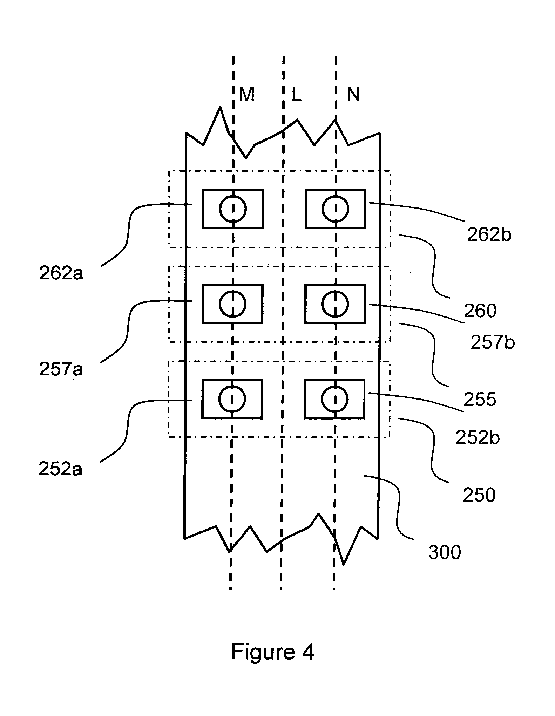

Figure 4 shows a schematic detailed view of the section of the PCB 300 on which the contact elements 252a, 252b, 257a, 257b, 262a, 262b are arranged in three pairs 250, 255, 260. The contact elements 252a, 252b, 257a, 257b, 262a, 262b are arranged in two lines defining a first contact element axis M and a second contact element axis N with the first contact element axis M and the second contact element axis N being parallel and equidistant to the device axis L. Each pair 250, 255, 260 comprises a first contact element 252a, 257a, 262a arranged in the first contact element axis M and a second contact element 252b, 257b, 262b arranged in the second contact element axis N.
In the following, reference is made to Figure 3 as well as to Figures 5 to Figure 8. Figure 5 shows a sectional view of the input unit, the line of vision being transversal to the device axis L, Figure 6 a cut-away top view (with the first housing component 170, the second housing component 180 and the control element 50 being cut away). Figure 7 is a sectional view, the line of vision being towards the cannula 20. Figure 8 is a detailed view of control element 50, the line of view being identical to Figure 7.
As seen from the figures, the control element 50 surrounds the first intermediate housing component 170 and the second intermediate housing component 180. Because the inner diameter of the control element 50 is substantially equivalent to the outer diameter D of the cylinder defined by the first intermediate housing component outer shell 192 and the second intermediate housing component outer shell 202, the first intermediate housing component outer shell (192) and the second intermediate housing component outer shell (202) define a sliding surface for the control element inner shell 51. Furthermore, the control element 50 exerts a radial force normal to the device axis L onto the first intermediate housing component 190 and onto the second intermediate housing component 200, the radial force fixing the first intermediate housing component 190 and the second intermediate housing component with respect to each other. Due to this arrangement, no further means such as additional snap-on connections are required for fixing the first intermediate housing component 190 and the second intermediate housing component 200 with respect to each other. However, additional fixing means may be provided additionally or alternatively in other embodiments. In combination with the snap-on connections (175, 195),the radial force exerted by control element 50 result in the first housing component 11, the second housing component 12, the first intermediate housing component 60 and the second intermediate housing component 70 being fixed with respect to each other. In this context it is worth noting that the snap-on connections 175, 195 are neither required nor designed to fix the first intermediate housing component 60 and the second intermediate housing component with respect to each other and/or with respect to the first housing component 11 and/or the second housing component 12 in a radial direction normal to the device axis L. However, this may be achieved by a modification of the snap-fits 175 and/or cutouts 195 in alternative embodiments.
In this specific embodiment, the control element inner shell additionally overlaps with a first housing component border area 174 and a second housing component border area 184.
As best seen from Figure 3, Figure 5 and Figure 6, a substantially U-shaped spring element 100 is arranged inside the housing 10 in symmetric alignment with the device axis L. As seen from Figure 5, the spring element 100 is supported by a first pocket 171 of the first housing component 170, the first pocket 171 serving as a first supporting element, and by a second pocket of the second housing component 12, the second pocket serving as a second supporting element 181. As shown in Figure 7 and Figure 8, an operation element 140 projects in radial direction from the circular control element inner shell 51. The operation element 140 is integral with the control element 50, the operation element having a base member 142 and a first extension member 144a and a second extension member 144b, both extension members 144a, 144b projecting in substantially transversal direction from the base member 142, resulting in an overall anchor-like shape of the operation element 140.
As best seen from Figure 6 and Figure 7, the intermediate housing components 190, 200 define an aperture 205, the aperture 205 being partly defined by each of the first intermediate housing component 190 and the second intermediate housing component 200. The base member 142 projects trough the aperture 205 into the housing 10 and is in part located between the elongated legs 102, 104 of the spring element 100, the elongated legs 102, 104 extending over the length of the aperture 205 in the direction defined by the device axis L.
As further visible in Figure 6, the edges of the intermediate housing components 190, 200 defining the aperture 205 are not straight, but show two pairs of fingers 206a, 206b, 207a, 207b, the fingers a guidance for the base member 142. Due to its contour, the aperture 205 serves as a guide and the base member 142 serves as motion restrictor. The interaction of the base member 142 and the aperture 205 restricts the motion of the control element 50 such that the control element 50 can be moved into the direction of the fist motion A along the device axis L only with the control element 50 being in its neutral elongation position 85 and can be moved into the direction of the second motion B from the neutral elongation position 85 into the first elongated operation position 90 and/or into the second elongated operation position 95 only with the control element 50 being in either of its selection position 70, 75, 80.
As best seen from Figure 6, the spring element 100 shows three salients 110, 112, 114. If the control element 50 is in either of the axial selection positions 70, 75, 80, the base member 142 is aligned with and projects through one of the salients 110, 112, 14, resulting in the spring element 100 exerting virtually no catching force onto the base member 142 which results in the selection positions 70, 75, 80 being stable. The spring element 100 exerts a significant catching force onto the base member 142 if the control element 50 is in an axial position in which the base member 142 is not aligned with and does not project through any of the salients 110, 112, 114, resulting in an unstable position of the control element 50. Accordingly, the control element 50 inherently tends to be in either of the selection positions 70, 75, 80 and the base member 142 serves as catching force transmitter.
If the control element 50 is in either of its selection position 70, 75 80 and is rotated from its neutral elongation position 85 into the first elongated operation position 90, either of the contact elements 252a, 257a, 262a may be operated, depending on the selection position 70, 75, 80. via the first extension member 144a of the operation element 140. In the same way, either of the contact elements 252b 257b, 262b may be operated via the second extension member 144b of the operation element 140 by rotating the control element 50 from its neutral rotational position 85 into the second elongated operation position 95.
If the control element 50 is rotated from its neutral elongation position 85 into either of the rotational operation positions 90, 95, the base member 142 serves as first restoring force transmitter. The contact of the base member 142 with the spring element 100 causes an elastic deformation of the spring element 100, resulting in a first restoring force being exerted by the spring element 100 onto the base member 142, the first restoring force resulting in a first restoring momentum exerted onto the control element 50. Additionally, the extension members 144a, 144b serve as a second restoring force transmitter, such that operation of either of the contact elements 252a, 252b, 257a, 257b, 262a, 262b results in a second restoring force being exerted onto extension member 144a or 144b, respectively. The second restoring force is a contact element restoring force and generated by the operated micro pushbutton 252a, 252b, 257a, 257b, 262a, 262b. The second restoring force results in an additional second restoring momentum exerted onto the control element 50.
While either of the first restoring force and the second restoring force would generally be sufficient, the combination is especially preferable for control element 50 being free from play and giving force feedback to the user.
In the context of an insulin administration device for the therapy of diabetes mellitus, the control element 50 is adapted for the simple and intuitive entry of numeric values, wherein the numeric values may especially represent therapy related and particularly carbohydrate amounts. Data entry is described with reference to Figure 2a, Figure 2b, and Figure 2c.
The second selection position 75 and the third selection position 80 are numeric value entry positions. In either of the first numeric value entry position 75 and the second numeric value entry position 80 a numeric value to be entered may be increased by rotating the control element 50 from the neutral elongation position 85 into the first elongated operation position 90 or may be decreased by rotating the control element 50 from the neutral rotational position 85 into the second elongated operation position 95. If the control element 50 is in the first numerical value entry position 75, rotation into the first elongated operation position 90 results in operating the contact element 257a, while rotation into the second elongated operation position 95 results in operating the contact element 257b. Similarly, if the control element 50 is in its second numerical value entry position 80, rotation into the first elongated operation position 90 results in operating the contact element 262a, while rotation into the second elongated operation position 95 results in operating the contact element 262b.
Carbohydrate amounts are preferably entered in carbohydrate exchange units which are used by many diabetics or in grams of carbohydrates. The administration device may be configured by the patient or the health care professional for carbohydrate amount entry in the desired units.
In order to easily enter both large and small carbohydrate amounts with sufficient resolution, a small carbohydrate increment I1 and a large carbohydrate increment 12 are provided. The first numeric value entry position 75 corresponds to the small carbohydrate increment I1 while second numeric value entry position corresponds to the large carbohydrate increment 12. Besides the entry of both large and small carbohydrate amounts, this functions enables the patient to coarsely enter a carbohydrate amount using the increment 12 and subsequent fine adjustment using the increment I1. Generally, each single rotation of the control element 50 from its neutral elongation position 85 into either of elongated operation positions 90, 95 increases/decreases the carbohydrate amount by an increment I1 or 12, respectively. However, a scroll function may also be implemented which allows to increase/decrease the carbohydrate amount as long as the control element 50 is held in the first elongated operation position 90 or the second elongated operation position 95 continuously. If carbohydrate amounts are entered in grams of carbohydrate, I1 may, e.g. correspond to 1 gram of carbohydrates and I2 may, e.g., correspond to 10 grams of carbohydrate.
After entering a carbohydrate amount, the input unit 50 is shifted to the selection position 70, the selection position 70 being an entry completing position. With the control element 50 being in the entry completing position 70, the entry may be confirmed and accepted by rotating the control element 50 from its neutral elongation position 85 into the first elongated operation position 90. By rotating the control element 50 from its neutral elongation position 85 into the second elongated operation position 95, the entry may be canceled. If the user does not modify the value nor confirms the entry within a certain time after of, e.g. 5 sec, the entry is preferably automatically canceled.
In the following, reference is made to Figure 9, Figure 9 showing a block diagram of the electronics module, the electronics module being mainly arranged on PCB 300 and fulfilling multiple functions. The core element of the electronics module is a controller 305, the controller 305 being realized as ASIC.
For scanning the rotational movement of the dose setting knob 25, an encoder 310 is provided, the encoder 310 detecting the rotation of the dose setting knob 25 and transferring a corresponding electrical signal to the controller 305 dependent on the direction of rotation. The encoder 310 comprises a set of came followers driven by a crank shaft, a set of came discs or the like operatively coupled to a corresponding set of electrical contacts. Similarly, optical encoders or magnetic encoders with magnets and hall sensors may be employed. The encoder 310 may, e.g., be of the design disclosed in WO 93/16743.
The Controller 305 is coupled with the display 30, the display 30 being a LCD and being adapted for showing numeric data and application specific data. When rotating the dose setting knob 25, an accumulated dose amount is computed by the controller 305 based on the signals generated by the encoder 310 and is displayed on the display 30 in real time while setting a dose amount. Similarly, carbohydrate amounts or other data which may be entered via the control element 50 are shown on the display 30 during entry. Besides the display 30, an acoustic buzzer 315 is provided especially for notification and indication purposes.
When actually administering an insulin dose by pressing down the dose setting knob 25, the accumulated dose is stored in the memory 320 together with a time stamp generated by clock circuit 325, the time stamp comprising a time of day portion and a date portion. Similarly, a carbohydrate amount entered via the control element 50 is stored in the memory 320 together with a time stamp after accepting the entry. Both the memory 320 and the clock circuit 325 are integral with the controller 305. The Memory 320 is realized as DRAM or FRAM and may be integral with the controller 305.

The electronics module further comprises a data interface 330 which usage will be described below in greater detail. In this exemplary embodiment, the data interface is a infrared communication interface as visible in Figure 3, according, e.g., to the IRDA standard. However other communication interfaces such an BLUETOOTH RF interface or a wired serial interface may be provided alternatively or additionally.
The electronics module further comprises a power supply in form of a replacable battery 35. The battery 35 is located under a removable cover 37 inside the housing 10 as shown in Figure 3. The electronics module changes from a power-saving standby state to regular operation whenever the dose setting knob 25 is extended or either of the contact elements 252a, 252b, 257a, 257b, 262a, 262b is operated.. It changes from regular operation to the standby state if no operation is being performed for a threshold time of, e.g. 2 minutes.
The administration of insulin and the entry of carbohydrate amounts are generally independent form each other and may accordingly be stored in memory 320 independently together with corresponding time stamps. So it is possible to only enter a carbohydrate amount without administering insulin. This situation may occur, e.g. when eating small snacks or when eating additional food during sportive activities. It is also possible to administer insulin without carbohydrate intake. This situation may occur when insulin is administered in order to compensate for undesirably increase blood glucose values.
In many cases, however, insulin administration and carbohydrate intake happen at virtually the same time. In this case, it is favorable to store both dose amount and carbohydrate amount together with a common time stamp. For this purpose, a time window of e.g., 2 minutes is provided. If both a dose amount is set and administered and an carbohydrate amount is entered within this time window, both the dose and the carbohydrate amount are stored in memory 320 with one common time stamp. This function is realized by a countdown timer starting with a countdown time, the countdown time representing the time window, and counting down to zero. The countdown timer is triggered by either of administering insulin or entering a carbohydrate amount, whichever happens first. If the other of the two operations is performed within the time window, the countdown timer is stopped and both carbohydrate amount and dose amount are stored together with a common timestamp. Otherwise only the carbohydrate amount or the administered dose is stored and a warning is given to the user. While this allows the patient to enter the carbohydrate amount and administer insulin in arbitrary order, a minor modification is possible to force the patient to enter the carbohydrate amount before insulin administration.
Because insulin doses required to compensate for food intake are directly correlated with the carbohydrate amount, the required dose amount can be automatically calculated based on the carbohydrate amount entered via the input unit 50. For this purpose, a dose calculator is provided by an algorithm on controller 305. Dose amounts are calculated and displayed based on a set of patient-specific and time-of-day dependent factors. The set of factors is preferably uploaded to the electronics module from an external device via the data interface 330 but may also be entered via the control element 50.
An external device which may communicate with the administration device via the data interface 330 may, e.g., be a Personal Digital Assistant (PDA), a cell phone or a dedicated diabetes management device which also may comprise a blood glucose meter. In many cases, the external device is a standard PC used for archiving and and/or analyzing therapy related data and/or for data upload purposes. Administration devices according the exemplary embodiment as well as blood glucose meters such as ACCU-CHEK® Compact plus or ACCU-CHEK® Aviva may be especially communicate with a PC via a device interface such as ACCU-CHEK® Smart Pix.

## Claims

1. Administration device for the self-administration of liquid drugs in adjustable doses, comprising:
a) a manually driven dosing mechanism, a drug reservoir, and a dose setting means (25), the dose setting means (25) being coupled to the dosing mechanism,
b) an electronics module,
c) a housing (10), the housing (10) comprising the dosing mechanism, the electronics module and the drug reservoir, the housing (10) further defining a device axis (L), the housing (10) being adapted to be held in a hand for drug administration,
d) a input unit, the input unit being coupled to the electronics module, the input unit comprising a control element (50), the control element (50) being different from the dose setting means (25), the control element (50) being arranged, at least in part, outside housing (10) and being movable by a first motion (A) with respect to the housing (10) into either of at least two selection positions (70, 75, 80), the control element (50) being further movable by a second motion (B) with respect to the housing (10) into either of at least two elongation positions (85, 90, 95), wherein the motion direction of the first motion (A) is different from the motion direction of the second motion (B).

2. Administration device according to Claim 1, **characterized in that** the first motion (A) is a linear motion (X), the linear motion being substantially parallel with the device axis (L).

3. Administration device according to Claim 2, **characterized in that** the second motion (B) is a rotation, the axis of rotation of the second motion (B) being substantially parallel to the device axis (L).

4. Administration device according to Claim 3, **characterized that** the control element (50) substantially has the form of a cylindrical ring, the ring surrounding a portion of the housing (10), wherein the housing outer shell (11) defines a sliding surface for the control element, such that the control element may slide on the housing outer shell (11) along the device axis (L) into either of the alt least two selection positions (70, 75, 80) and may rotate about the device axis (L) into either of the at least two elongation positions (85, 90, 95).

5. Administration device according to either of the previous claims, **characterized in that** at least one of the at least two selection positions (70, 75, 80) is a catching position (70, 75, 80).

6. Administration device according to either of the previous claims, **characterized in that** the first motion (A) of control element (50) is enabled only if the control element (50) is in a defined neutral elongation position (85).

7. Administration device according to either of the previous claims, **characterized in that** the second motion (B) of control element (50) is enabled only if the control element (50) is in either of the at least two selection positions (70, 75, 80)

8. Administration device according to Claim 6 or Claim 7, **characterized in that** the input unit comprises a the guide, the guide restricting the motion of the control element.

9. Administration device according to either of the previous claims, **characterized in that** moving the control element (5) into either of at least two elongated operation positions (90, 95), the elongated operation positions (90, 95) being different from a neutral elongation position (85), results in a fist restoring force being exerted onto the control element (50).

10. Administration device according to either of the previous claims, **characterized in that** the control element (50) comprises an operation element (140), the operation element (140) projecting into the housing (10).

11. Administration device according to either of the previous claims, **characterized in that** the input unit comprises contact elements (252a, 252b, 257a, 257b, 262a, 262b), wherein each contact element (252a, 252b, 257a, 257b, 262a, 262b) can be selected by moving the control element (50) in into the selection position (70, 75, 80) corresponding to the contact element (252a, 252b, 257a, 257b, 262a, 262b) and wherein a selected contact element (252a, 252b, 257a, 257b, 262a, 262b) can be operated by moving the control element (50) from a neutral elongation position (85) into an elongated operation position (90, 95) while keeping the selection position (70, 75, 90).

12. Administration device according to Claim 11, **characterized in that** at least one of the selection positions (70, 75, 90) selects a pair (250, 255, 26) of contact elements (252a, 252b, 257a, 257b, 262a, 262b), such that a first contact element (252a, 257a, 262a) of a selected pair (250, 255, 260) of contact elements (252a, 252b, 257a, 257b, 262a, 262b) can be operated by moving the control element (50) into a first elongated operation position (90) and **in that** a second contact element (252b, 257b, 262b) of a selected pair (250, 255, 260) of contact elements (252a, 252b, 257a, 257b, 262a, 262b) can be operated by moving the control element (50) into a second elongated operation position (90).

13. Administration device according to either of Claim 11 or Claim 12, **characterized in that** operating a contact element (252a, 252b, 257a, 257b, 262a, 262b) results in a second restoring force being exerted onto the control element (140).

14. Administration device according to either of the previous claims, **characterized in that** the drug reservoir is a cylindrical cartridge, the cartridge axis being substantially parallel with the device axis (L), the cartridge comprising a plunger, the plunger being displacable in a direction defined by the cartridge axis, and **in that** the dosing mechanism comprises a piston rod, the piston rod being coaxially aligned with the cartridge axis and being in contact with the plunger, such that the plunger is displaced by a displacement distance Δ in a direction defined by the cylinder axis if the piston rod is displaced by a displacement distance A, wherein the displacement distance Δ is controlled by a dose setting means (25).

15. Administration device according to either of the previous claims, **characterized in that** at least one of the selection positions (70, 75, 80) is a numeric value entry position (70, 80), such that moving the control element (50) from a neutral elongation position (85) into a first elongated operation position (90) increases a numeric value to be entered by at least one increment I and **in that** moving the control element (50) from a neutral elongation position (85) into a second elongated operation position (90) decreases a numeric value to be entered by at least one increment I, wherein the control element (50) is in the at least one numeric value entry position (70, 80).

16. Administration device according to Claim 15, **characterized in that** at least two of the selection positions (70, 75, 80) are numeric value entry position (70, 80), such that the increment I equals a first increment I 1 if the control element 50 is in a first numeric value entry position (70) and that the increment I equals a second increment I2 if the control element 50 is in a second numeric value entry position, wherein the first increment I1 is different from the second increment 12.

17. Administration device according to either of the previous claims, **characterized in that** the electronics module comprises memory (320), the memory (320) being adapted to store data entered via the input unit.

18. Administration device according to claim 17, **characterized in that** the electronics module comprises a data interface (330), the data interface (330) being adapted to download the data stored in the memory (320) to an external device.

19. Administration device according to either of the previous claims, **characterized in that** the electronics module comprises a dose calculator, the dose calculator being adapted to calculate a dose amount of the liquid drug based, at least in part, on data entered via the input unit.

20. Administration device according to either of the previous claims, **characterized in that** the electronics module comprises a display (30), the display (30) being adapted to show data entered via the input unit.
